# EUROPÄISCHE PATENTANMELDUNG

(11) **EP 1 707 623 A1**
(43) Veröffentlichungstag der Anmeldung: **04.10.2006**
(21) Anmeldenummer: 05007157.0
(22) Anmeldetag: 01.04.2005
(51) Int. Cl.: C12N 9/22, C12Q 1/68

(54) **Reverse Transkription und Amplifikation von RNA bei simultaner Degradierung von DNA**

(71) Anmelder: QIAGEN GmbH, 40724 Hilden (DE)
(72) Erfinder: Korfhage, Christian, 40764 Langenfeld (DE); Paist, Ralf, 40229 Düsseldorf (DE); Loeffert, Dirk, 40589 Düsseldorf (DE)
(74) Vertreter: Leidescher, Thomas

(57) **Zusammenfassung**

Die vorliegende Erfindung betrifft ein Verfahren zur reversen Transkription und Amplifikation von RNA, das dadurch gekennzeichnet ist, daß in einem Reaktionsgefäß die RNA-Reaktion und eine Degradierung von vorhandener doppelsträngiger oder einzelsträngiger DNA erfolgen. Ein Abbau von RNA oder eines DNA7RNA-Hybridmoleküls unterbeleibt jedoch. Degradierung der DNA-Kontamination und die Reaktion der RNA werden zur selben Zeit bei der selben Temperatur und in ein und demselben Reaktionsansatz bzw. Reaktionsgefäß durchgeführt.

## Beschreibung

Die vorliegende Erfindung betrifft ein Verfahren zur Behandlung von RNA, insbesondere ein RNA-Reaktionsverfahren, sowie Kits zur Durchführung einer cDNA Synthesereaktion und zur Durchführung einer 1-step-RT-PCR Reaktion.

Viele Techniken in der Molekularbiologie führen zur Analyse von Ribonukleinsäuren (RNA). Um RNA analysieren zu können, muß diese zuvor von allen inhibitorischen und kontaminierenden Substanzen gereinigt werden. So können z.B. Kontaminationen von genomischer Desoxyribonukleinsäure (DNA) inhibitorisch wirken oder zu falsch positiven Ergebnissen führen. Einige Techniken molekularer RNA-Analytik starten mit der reversen Transkription von RNA in cDNA. cDNA ist in Struktur und Sequenz sehr ähnlich bis identisch zu genomischer DNA. Daher kann eine Kontamination von genomischer DNA zu falschen Ergebnissen führen, wenn cDNA analysiert werden soll (z.B. photometrische Bestimmung der cDNA-Menge oder deren Quantifizierung durch PCR).

Um RNA sicher analysieren zu können, ist es daher notwendig, alle störenden anderen Nukleinsäuren, wie z.B. genomische DNA, vor einer entsprechenden Analyse entweder von der RNA abzutrennen oder in ihre einzelnen Bausteine zu zerlegen. Ein seit langem angewandtes Trennungsverfahren für DNA und RNA ist die sog. Dichtegradientenzentrifugation. Standardsubstanzen für die Dichtegradientenzentrifugation sind Cäsiumchlorid (CsCl) und Saccharose. Für CsCl stellt sich z.B. je nach Dichte der Ausgangslösung während der Zentrifugation im Gleichgewichtszustand ein Dichtegradient ein, in dem sich jedes Makromolekül in der seiner eigenen Dichte entsprechenden Zone im Gradienten einordnet. Um die sich nach der Zentrifugation einstellenden Nukleinsäurebanden in dem Zentrifugationsgefäß sichtbar zu machen, wird der CsCI-Lösung Ethidiumbromid zugegeben, das sich in die Nukleinsäure einlagert und im UV-Licht fluoresziert. Mit dieser Methode lassen sich einzelne DNA-Bruchstücke, die aufgrund der eng zusammenliegenden Sedimentationsraten dieser Bruchstücke ansonsten sehr schwer voneinander trennbar sind, sicher abtrennen. Bei der CsCl-Dichtegradientenzentrifugation verwendet man üblicherweise Gradienten mit Dichtewerten zwischen 1,0 und 1,9 g/ml. Da die Schwebedichte der RNA üblicherweise größer als 1,9 g/ml ist, schlägt sich die RNA bei einer Gleichgewichtszentrifugation (auch isopyknische Zentrifugation genannt) in einem Gradienten, dessen Dichte-Obergrenze bei 1,9 g/ml liegt, am Boden des Probengefäßes nieder, während alle anderen Molekülarten (und damit auch die DNA) jeweils Banden innerhalb des Gradienten ausbilden. Entsprechende Trennungsoperationen führen daher zu einer guten Auftrennung von RNA und DNA. Das Dichtegradientenzentrifugations-Verfahren ist jedoch aufgrund der eingesetzten Chemikalien relativ teuer, apparativ sehr aufwendig und auch sehr zeitintensiv (bis sich ein Gleichgewicht eines üblichen CsCI-Gradienten eingestellt hat, sind mit den meisten Rotoren lange Zentrifugenläufe von bis zu 2 Tagen notwendig).

Deshalb wurde bei der Gewinnung von RNA dazu übergegangen, während oder nach der RNA-Präparation dem Versuchsansatz eine DNase oder mehrere DNasen zuzugeben, um DNA Kontaminationen enzymatisch abzubauen. Auf dem Markt erhältliche Systeme (sog. "Kits") für diesen Zweck werden von der Firma QIAGEN, Hilden, Deutschland unter den Bezeichnungen *"RNeasy Micro Kit"* und *"RNeasy Fibrous Tissue Kit"* und von der Firma Promega, Madison/WI, USA, unter der Bezeichnung *"SV Total RNA Isolation System* angeboten. Allerdings führen diese RNA-Präparationsmethoden nicht zur Isolierung reiner RNA. Die gewonnene RNA liegt vielmehr als Material vor, das noch in unterschiedlichem Maße mit genomischer DNA, Salzen, Inhibitoren etc. verunreinigt ist. Für manche Anwendungen mag der mit den o.g. Kits erreichbare Reinigungsgrad ausreichend sein, für einige andere Einsatzgebiete (z.B. RT-PCR) ist dies jedoch nicht der Fall.

Auch wird noch ein Verfahren zur RNA-Reinigung eingesetzt, bei dem chromatographische Methoden (z.B. Ionenaustausch-Chromatographie, Oligo-dT-Chromatographie) verwendet werden, um RNA nochmals anzureichern und die Menge an DNA zu reduzieren. Mit dieser Methode ist es allerdings nicht möglich, ribosomale RNA aufzureinigen.

Schließlich beschreibt die US-Patentanmeldung Nr. 20020042052 noch ein Verfahren zur Entfernung von Nukleinsäureverunreinigungen aus einem Ansatz für Amplifikationsreaktionen. Dabei wird eine thermolabile DNase eingesetzt, die immer vor der eigentlichen Amplifikationsreaktion unerwünschte doppelsträngige DNA in dem Amplifikationsansatz abbaut. Aufgrund ihrer Thermolabilität wird die eingesetzte DNase spätestens bei der ersten Temperaturerhöhung auf über 90°C bei der PCR-Reaktion irreversibel inaktiviert. Die PCR-Reaktion kann daher erst dann begonnen werden, wenn die DNase-Reaktion abgeschlossen ist. Eine Gleichzeitigkeit der DNA-Abbaureaktion und einer RNA-Reaktion ist daher bei dem aus der genannten amerikanischen Offenlegungsschrift bekannten Verfahren nicht gegeben.

Auch alle diese neueren Verfahren sind jedoch teilweise zeitaufwendig, kostenintensiv und können gegebenenfalls bei gleichzeitiger Bearbeitung mehrerer RNA-Präparationen zu Kreuzkontaminationen führen. Auch gilt für alle der oben beschriebenen vorbekannten Verfahren, daß dabei nicht simultan mit der RNA-Reaktion oder RNA-Analyse eine DNA Degradierung erfolgt, sondern immer vor der eigentlichen RNA-Reaktion oder RNA-Analyse der DNA Abbau durchgeführt wird.

Der vorliegenden Erfindung liegt somit die Aufgabe zugrunde, ein Verfahren für die RNA-Analytik zur Verfügung zu stellen, das die Nachteile der oben beschriebenen bekannten Verfahren nicht aufweist. Das neue Verfahren soll kostengünstig und wenig zeitaufwendig sein und den apparativen Aufwand in Grenzen halten.

Diese Aufgabe löst die Erfindung durch das Verfahren gemäß des unabhängigen Anspruchs 1 und der Kits gemäß der unabhängigen Ansprüche 15 und 16. Weitere vorteilhafte Ausgestaltungen, Aspekte und Details der Erfindung ergeben sich aus den abhängigen Ansprüchen, der Beschreibung, den Beispielen und der Zeichnung.

Die vorliegende Erfindung betrifft somit ein RNA-Reaktionsverfahren, das dadurch gekennzeichnet ist, daß im selben Reaktionsgefäß eine RNA-Reaktion und eine Degradierung von vorhandener DNA erfolgen. Vorzugsweise finden die RNA-Reaktion und die Degradierung vorhandener DNA gleichzeitig statt. Dies hat den großen Vorteil, daß mit dem Beginn einer RNA-Reaktion nicht mehr gewartet werden muß, bis die in dem Reaktionsansatz vorhandene, unerwünschte DNA vollständig oder zumindest soweit abgebaut ist, daß sie die RNA-Reaktion oder die anhängige Analysereaktion nicht mehr stört. Auch wird durch das erfindungsgemäße Verfahren die Gefahr vermindert, durch häufiges Öffnen des Reaktionsgefäßes Verunreinigungen in den Reaktionsansatz einzuschleppen.

Auch kann bei dem erfindungsgemäßen Verfahren die RNA-Reaktion einerseits und der DNA-Abbau bei der selben Temperatur erfolgen. Die Temperatur kann dabei z.B. in einem Bereich von 10 bis 80°C, vorzugsweise 20 bis 70°C, insbesondere 20 bis 60°C, liegen.

Die Erfindung verknüpft somit erstmals die DNA-Dekontaminierung einer Probe mit der Reaktion der RNA in einem simultanen Prozeß, d.h. die DNA-Dekontaminierung einerseits und die RNA-Reaktion bzw. die RNA-Analyse andererseits laufen entweder nacheinander oder gleichzeitig bzw. nebeneinander in ein und demselben Reaktionsgefäß ab. Typische RNA-Analyse-Reaktionen sind z.B. reverse Transkription, one-step RT-PCR (Reverse Transkription Polymerasekettenreaktion in einem Schritt) oder Markierungsreaktionen der RNA.

Die vorliegende Erfindung gewährleistet somit eine Degradierung (d.h. den Abbau) unerwünschter, einzelsträngiger und/oder doppelsträngiger DNA (z.B. genomischer DNA; zirkulärer oder linearer Plasmid-DNA) gleichzeitig mit Reaktionen, die RNA als Analyten enthalten (z.B. reverse Transkription, siehe oben). Die Degradierung der einzelsträngigen bzw. doppelsträngigen DNA erfolgt innerhalb der Reaktion, die RNA als Analyten enthält, durch eine Desoxyribonuklease (DNase) oder mehrere DNasen, die spezifisch DNA hydrolytisch spaltet bzw. spalten. Ferner sind diese DNasen dadurch charakterisiert, daß eine Spaltung von RNA oder DNA, die als RNA-DNA Hybrid vorliegt, nicht oder in nur sehr geringem Maße erfolgt. Mit der vorliegenden Erfindung ist es erstmals möglich, daß in Gegenwart z.B. einer cDNA-Synthese-Reaktion, bei der eine RNA in einzelsträngige DNA umgeschrieben wird, simultan eine doppelstrangspezifische DNase zur Degradierung doppelsträngiger DNA eingesetzt wird, wobei die gerade im Syntheseprozeß entstehende, einzelsträngige cDNA nur sehr wenig oder gar nicht degradiert wird. Die erfindungsgemäß einsetzbaren DNasen können thermostabil oder thermolabil sein.

Wie oben bereits erwähnt, betrifft die vorliegende Erfindung die Verknüpfung von Verringerung der DNA-Kontamination einerseits und Reaktion der RNA andererseits in einem simultanen Prozeß. Wichtig dabei sind die Reaktionsbedingungen, die gleichermaßen eine DNA-Degradierung wie auch die Reaktion mit der RNA erlauben, wobei die DNA-Dekontaminierung z.B. mittels einer DNase durchgeführt wird.

Eine "DNA-Kontamination'" oder "DNA-Verunreinigung" in einem RNA-Isolat ist definiert als jegliches doppelsträngige oder einzelsträngige Desoxyribonukleinsäure-VLolekül, das unterschiedlicher Herkunft sein kann und zusammen mit der RNA als unerwünschtes Molekül isoliert wird. Doppelsträngigkeit der DNA kann auch dadurch entstehen, daß eine Einzelstrang-DNA durch Selbst-Hybridisierung zurückgefaltet vorliegt und somit zumindest zeitweise doppelsträngig vorkommt.

Die doppelsträngige DNA (dsDNA) kann dem ursprünglichen biologischen Material entstammen, aus dem auch die RNA isoliert worden ist. Somit kann diese nukleärer, plastidärer oder mitochondrialer Natur sein. Die dsDNA kann auch aus einer externen Quelle auf biologischem Wege auf das ursprüngliche biologische Material übertragen worden sein, sei es durch Infektion, Transformation, Fusion, Inkorporation oder ähnliches und kann somit z.B. viraler, prokaryontischer oder eukaryontischer Herkunft sein. Zudem kann die DNA auch auf unnatürlichem Wege auf das ursprüngliche biologische Material übertragen worden sein, wie z.B. durch Elektroporation, Transformation, Transfektion oder andere Techniken. Es kann sich dabei um genomische DNA, Plasmid-DNA, doppelsträngige Oligonukleotide oder andere Formen doppelsträngiger DNA handeln. Darüber hinaus kann die doppelsträngige DNA auch während oder nach der RNA-Isolierung in die RNA-Präparation eingeschleppt worden sein.

Als "Reaktion der RNA" wird z.B. definiert:
(1) Jegliche Form der Veränderung der RNA, wie z.B. eine Degradierung (ein Abbau), eine Markierung, Verlängerung, Modifizierung oder ähnliches. Dabei kann die RNA als einzelsträngiges, doppelsträngiges oder als Hybridmolekül (z.B. RNA-DNA-Hybrid) vorliegen;
   Bei der Degradierung handelt es sich vorzugsweise um eine spezifische Degradierung, bei der RNA selektiv mittels z.B. Ribozym, RNase H und/oder siRNA degradiert wird. Als RNase H kann auch ein beispielsweise durch Mutation oder chemische Modifikation gewonnenes RNase H⁻ Enzym verwendet werden.
(2) jede Form von Umsetzung, bei der RNA als Matrize für Polymerase-Reaktionen verwendet wird, wie z.B. (a) reverse Transkription oder (b) Transkription durch RNA-Polymerasen oder ähnliches;
   zu (a): Reverse Transkription kann dabei durchgeführt werden durch mutierte und durch nicht mutierte RNA-abhängige DNA-Polymerasen, wie z.B. Reverse Transkriptasen von Viren, Retrotransposons, Bakterien etc., aber auch Reverse Transkriptasen, die so mutiert sind, daß die RNase H Funktion der Reversen Transkriptase eingeschränkt wurde (z.B. VIMLV-RT RNase H minus). RNA-abhängige DNA-Synthese (reverse Transkription) kann auch durchgeführt werden durch Enyzme, die durch Mutation oder veränderte Reaktionsbedingungen eine veränderte Nukleinsäue-Abhängigkeit aufweisen und so die Funktion der RNA-abhängigen DNA-Polymerase erhalten. Als Beispiel sei hier die Tth-DNA-Polymerase genannt, die DNA-abhängig ist und durch Verwendung veränderter Reaktionsbedingungen auch RNA als Matrize verwenden kann.
   zu (b): Eine RNA-Polymerase Reaktionen ausgehend von RNA als Matrize kann durchgeführt werden durch mutierte wie durch nicht mutierte RNA-abhängige RNA-Polymerasen von z.B. Viren, Prokaryonten oder Eukaryonten. RNA-abhängige RNA-Synthese kann auch durchgeführt werden durch Enzyme, die durch Mutation oder veränderte Reaktionsbedingungen eine veränderte Nukleinsäue-Abhängigkeit aufweisen und so die Funktion der RNA-abhängigen RNA-Polymerase erhalten. Als Beispiel sei hier ein RNA-Amplifikationsverfahren, das eine T7-RNA Polymerase und RNA als Matrize verwendet, genannt (EP 1 056 884);
(3) jede Form der Umsetzung, bei der die RNA als Katalysator dient, wie z.B. Ribozyme etc.;
(4) jede Form einer Bindungsreaktion. An der Bindungsreaktion können verschiedene Bindungspartner beteiligt sein, wobei mindestens ein Partner RNA ist. Es sind folgende Bindungsreaktionen denkbar, wie z.B. RNA-RNA, RNA-DNA, RNA-PNA (weitestgehend als Hybridisierung bekannt), RNA-Antikörper-Reaktionen, RNA-Aptamer-Reaktionen, Erkennungsreaktionen der RNA mit anderen Molekülen, wie z.B. Antibiotika, oder ähnliches;
(5) jede Form von zusammengesetzten Reaktionen, bei denen die oben genannten Reaktionen (1) bis (4) ein Bestandteil der Gesamtreaktion sind, wie z.B. lineare RNA-Amplifikationsreaktionen (z.B. Eberwine, Epiclone, Nugen), exponentielle RNA-Amplifikationsverfahren (z.B. NASBA, TMA) oder andere Amplifikationsverfahren (z.B. SAGE, RT-PCR, RCA).

Wenn in Bezug auf die vorliegende Erfindung von RNA als "Reaktionsteilnehmer" bzw. von einem "RNA-Reaktionsverfahren", einer "RNA-Reaktion" oder einer "RNA-Analyse" gesprochen wird, dann bedeutet dies, daß die RNA bei einer entsprechenden Reaktion bzw. Analyse zwar beteiligt ist, aber nicht unbedingt verändert werden muß. Auch bei Reaktionen, aus denen die RNA unverändert hervorgeht (wie z.B. wenn die RNA als Katalysator oder Matrize wirkt), wird die RNA gemäß der vorliegenden Erfindung als "Reaktionsteilnehmer" bezeichnet bzw. spricht man von einem "RNA-Reaktionsverfahren", einer "RNA-Reaktion" oder einer "RNA-Analyse".

Wenn in Zusammenhang mit der vorliegenden Erfindung von "gleichzeitig" oder "Gleichzeitigkeit", "nebeneinander", "simultan" o.ä. gesprochen wird, so ist darunter zu verstehen, daß die DNA-Degradierung und die Reaktion bzw. Analyse der RNA im selben Reaktionsgefäß erfolgen. Degradierung der DNA-Kontamination und die Reaktion bzw. Analyse der RNA werden daher zur selben Zeit und in ein und demselben Reaktionsansatz durchgeführt. Durch die Gleichzeitigkeit soll zum Ausdruck gebracht werden, daß die Reaktion bzw. Analyse der RNA und die Degradierung der DNA-Kontamination zeitgleich im selben Reaktionsgefäß und unter den selben Reaktionsbedingungen ablaufen.

Vorteilhaft bei dem erfindungsgemäßen Verfahren, insbesondere gegenüber dem aus der US 20020042052 bekannten Verfahren, ist auch, daß das erfindungsgemäße Verfahren bei einer einheitlichen Temperatur ablaufen kann, d.h., daß die RNA-Reaktion und die DNA Degradierung bei der selben Temperatur ablaufen können. Außerdem ist es gegenüber dem aus der US 20020042052 bekannten Verfahren günstig, daß es bei dem erfindungsgemäßen Verfahren nicht notwendig ist, nach dem DNA Abbau mittels DNase das Reaktionsgefäß nochmals zu öffnen und ein neues Enzym (das bei einer Erwärmung auf über 90°C möglicherweise irreversibel geschädigt werden würde) zuzugeben, da dadurch unnötige Kontaminationen vermeiden werden können.

Gemäß der vorliegenden Erfindung ist die Reaktion der RNA begrenzt durch die Reaktionsbedingungen, die nicht nur die Reaktion der RNA bestimmen, sondern auch simultan die Degradierung der DNA-Kontamination ermöglichen sollen. Dies heißt nicht, daß für die Reaktion der RNA und die Degradierung der DNA die jeweils optimalen Reaktionsbedingungen eingesetzt werden, sondern daß die Bedingungen für die Reaktion der RNA und die Degradierung der DNA durchaus aneinander angepaßt werden können.

Eine "Desoxyribonuklease" oder kurz "DNase'" ist hier definiert als ein Molekül, das spezifisch einzelsträngige und/oder doppelsträngige DNA hydrolytisch spaltet (degradiert). Diese DNase ist dadurch charakterisiert, daß ein Abbau bzw. eine Spaltung von RNA oder DNA, die als RNA-DNA Hybrid vorliegt, nicht oder in nur vemachlässigbar geringem Maße erfolgt. Ferner ist die DNase dadurch gekennzeichnet, daß DNA endonukleolytisch und/oder exonukleolytisch abgebaut werden kann. DNasen sind entweder sequenzspezifisch oder auch nicht. Beide Varianten können im Rahmen der vorliegenden Erfindung eingesetzt werden. Ebenso ist es möglich, thermolabile und/oder thermostabile DNasen einzusetzen.

Unter "Abbau" bzw. "Degradierung" oder "Degradation" der DNA ist zu verstehen, daß der Abbauvorgang in jedem Fall so weit voranschreitet, daß die DNA nur noch geringe oder keine störenden Einflüsse mehr auf die Reaktion(en) der RNA oder die Nachfolge-Anwendungen zeigt. Ein Abbau bzw. eine Degradierung kann, muß aber nicht eine vollständige Zerlegung doppelsträngiger DNA in deren Einzelbausteine (Nukleotide) bedeuten.

Gemäß eines bevorzugten Aspekts betrifft die vorliegende Erfindung ein RNA-Reaktionsverfahren, das dadurch gekennzeichnet ist, daß unter der Voraussetzung, daß die verwendete Reverse Transkriptase eine RNase H-Aktivität aufweist (RNase H+), vor dem Schritt, in dem eine RNA-Reaktion und eine Degradierung von vorhandener DNA im selben Reaktionsgefäß zur gleichen Zeit und bei der selben Temperatur erfolgen, durch eine reine Temperaturänderung ein weiterer Schritt durchgeführt wird, bei welchem die Temperatur soweit verringert wird, dass die Degradierung von DNA erfolgt, die RNase H-Aktivität der Reversen Transkriptase jedoch unterdrückt wird.

In der Zeichnung zeigen:
- Fig. 1: ein Foto eines Agarose-Gels, welches das Ergebnis einer Elektrophorese gemäß Beispiel 1 zeigt;
- Fig. 2: ein Balkendiagramm, aus welchem die Ergebnisse eines Versuchs zur möglichen Beeinflussung der Reverse Transkriptase-Reaktion durch diverse Nucleasen ersichtlich sind (Beispiel 2);
- Fig. 3: ein Foto eines Agarose-Gels, welches das Ergebnis einer Elektrophorese gemäß Beispiel 3 zeigt;
- Fig. 4: ein Diagramm, das die in Tabelle 1 genannten Ergebnisse in graphischer Form zeigt; und
- Fig.5: ein Balkendiagramm, das den Einfluß pankreatischer DNase 1 auf ein cDNA- und gDNA-Signal gemäß Beispiel 7 zeigt.

Folgende Enzyme können zur Degradierung von genomischer DNA verwendet werden, insbesondere in Reverse Transkriptase-Reaktionen:
1) Sequenzunabhängige Endonucleasen: Diese Endonucleasen können ein natives Enzym sein (Isolat aus einem Organismus) oder aus gentechnisch veränderten Organismen (GVOs) hergestellt werden. Ein Beispiel für eine sequenzunabhängige Endonucleasen ist die DNase I, welche erfindungsgemäß besonders bevorzugt zum Abbau von gDNA eingesetzt wird;
2) Sequenzspezifische Endonucleasen: Diese Endonucleasen können ein natives Enzym sein (Isolat aus einem Organismus) oder aus gentechnisch veränderten Organsimen (GVOs) hergestellt werden. Beispiele für sequenzspezifsche Endonucleasen sind die Enzyme Alu I oder Hae III. Auch Mischungen von sequenzspezifischen DNA-Nucleasen können verwendet werden.
3) Kombinationen von sequenzspezifschen Endonucleasen und sequenzunspezifschen Exonucleasen oder sequenzunspezifschen Endonucleasen

Die DNase(n) werden üblicherweise derart eingesetzt, daß der Reaktionsansatz etwa 0,01 bis etwa 100 U an Enzymaktivität enthält, vorzugsweise etwa 0,05 bis etwa 20 U, mehr bevorzugt etwa 0,1 bis etwa 10 U. Nach internationaler Übereinkunft entspricht die als 1 U (unit, Enzymeinheit) angegebene Enzymaktivität der Menge an Enzym, die benötigt wird, um pro Minute bei 25°C unter optimalen Bedingungen 1 umol Substrat umzusetzen.

Die Degradierung der genomischen DNA wird im Falle einer PCR in Anwesenheit einer Reversen Transkriptase durchgeführt. Taugliche Reverse Transkriptasen sind beispielsweise Reverse Transkriptasen aus Retroviren, wie z. B. HIV, AMV, EIAV, MMLV, Omniscript® (QIAGEN GmbH, Hilden Deutschland), Sensiscript® (QIAGEN GmbH, Hilden Deutschland) etc. oder auch aus Retrotransposons. Die Reversen Transkriptasen können in ihrer Aminosäuresequenz dem Ursprungsorganismus entsprechen oder sie können auch Abweichungen davon haben, wie z.B. Veränderungen, die zum Verlust der RNase H Aktivität führen, die Prozessivität verändern oder die Thermostabilität des Enzyms beeinflussen. Es können auch DNA-Polymerasen verwendet werden, die ursprünglich keine oder nur eine geringe Reverse Transkriptase-Aktivität aufweisen und durch Verwendung geeigneter Reaktionsbedingungen oder durch Mutationen als Reverse Transkriptase verwendet werden können (z.B. rTth-Polymerase).

Die wäßrige Pufferlösung, in der die Degradierung von genomischer DNA in Gegenwart einer Reversen Transkriptase abläuft, enthält mindestens:
1) eine Nuklease (wie oben beschrieben);
2) eine Reverse Transkriptase (wie oben beschrieben);
3) eine Puffersubstanz, die den pH-Wert des Versuchsansatzes puffert;
4) einen pH-Wert zwischen 6 und 10, besonders bevorzugt zwischen 7 und 9; und
5) bivalente Kationen, die eine Reverse Transkriptase-Reaktion und die enzymatische Degradierung von genomischer DNA unterstützen, wie z.B. Mg²⁺(in einem Konzentrationsbereich zwischen 0,1 und 50 mM), Mn²⁺ (in einem Konzentrationsbereich zwischen 0,01 und 10 mM), oder Ca 2⁺ (in einem Konzentrationsbereich zwischen 0,01 und 50 mM).

Der Reaktionsansatz kann auch noch andere Enzyme oder Salze enthalten. So kann z.B. auch eine hitzestabile DNA-Polymerase enthalten sein.

Die Reaktionstemperatur kann z.B. zwischen 10 und 70°C liegen, bevorzugt zwischen 15°C und 60°C, ganz besonders bevorzugt zwischen 20°C und 50°C.

Die vorliegende Erfindung betrifft des weiteren einen Kit zur Durchführung einer cDNA Synthesereaktion, insbesondere zur Durchführung eines Verfahrens gemäß einem der Ansprüche 1 bis 12, wobei der Kit mindestens eine reversen Transkriptase, eine DNA abhängige Nuclease und einen Reaktionspuffer zur Durchführung einer cDNA Synthese und einer DNA Degradierung in einem Gefäß enthält sowie einen weiteren Kit zur Durchführung einer 1-step-RT-PCR Reaktion, insbesondere zur Durchführung eines Verfahrens gemäß einem der Ansprüche 1 bis 12, wobei dieser Kit mindestens eine reverse Transkriptase, eine DNA abhängige Nuclease, eine thermostabile DNA Polymerase und einen Reaktionspuffer zur Durchführung einer 1-step-RT PCR Reaktion und einer DNA Degradierung in einem Gefäß enthält.

Die Erfindung wird nachfolgend anhand der Beispiele näher erläutert.

### Beispiel 1

Es wurden jeweils 1 µg hochmolekulare DNA und 1 µg Total-RNA aus HeLa-Zellen gemischt, um in einer Reverse-Transkriptase Reaktion eingesetzt zu werden. Die Reverse Transkriptase-Reaktion fand dabei in einem wäßrigen Milieu statt, das einen Oligo-dT-Primer, dNTPs, einen RNase Inhibitor, einen Puffer (Buffer RT aus dem Omniscript RT Kit der QIAGEN GmbH, Hilden, Deutschland) für die reverse Transkription und eine reverse Transkriptase (Omniscript® , Marke der QIAGEN GmbH, Hilden, Deutschland) enthielt. Zudem wurden unterschiedliche doppelstrangspezifische DNasen zugegeben:
(1) Alu 1 Restriktionsendonuklease in einer Menge von 10 U (erhältlich von Roche, Nlannheim, Deutschland);
(2) Hae III Restriktionsendonuklease in einer Menge von 10 U (erhältlich von Roche, Mannheim, Deutschland).
(3) RNase-freie DNase 1 in einer Menge von 10 U (erhältlich von Roche, Mannheim, Deutschland);
(4) Alu I Restriktionsendonuklease in einer Menge von 10 U (erhältlich von Roche, Mannheim, Deutschland) und Hae III Restriktionsendonuklease in einer Menge von 10 U (erhältlich von Roche, Deutschland).

Einem weiteren Reaktionsansatz wurde keine DNase hinzugefügt. Dieser Ansatz diente als Kontrolle. Das Reaktionsgemisch wurde für 1 Stunde bei 37°C inkubiert und anschließend (A) mittels PCR auf cDNA-Degradierung analysiert und (B) auf einem Agarose-Gel (1,2 %) auf RNA-Integrität und DNA-Degradierung getestet.

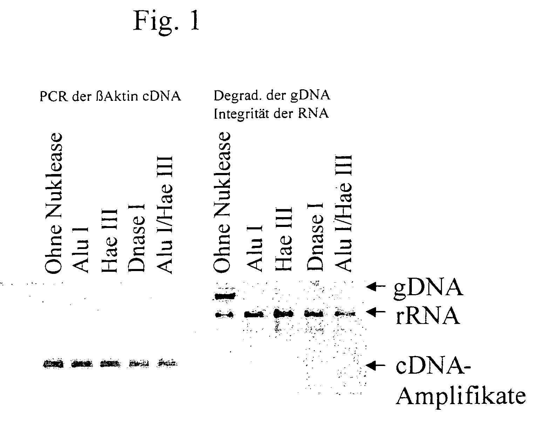

Das Ergebnis ist in Fig. 1 gezeigt.

### Beispiel 2

Jeweils 1 ng Total-RNA aus HeLa-Zellen wurden mit 1 µg einer 0,2-9,5 kB RNA-Leiter (Invitrogen) gemischt, um in einer Reverse-Transkriptase Reaktion eingesetzt zu werden. Die Reverse-Transkriptase Reaktion wurde dabei in einem wäßrigen Milieu durchgeführt, das einen Oligo-dT-Primer, dNTPs, RNase Inhibitor, einen Puffer (Buffer RT aus dem Omniscript RT Kit der QIAGEN GmbH, Hilden, Deutschland) für die reverse Transkription und eine Reverse Transkriptase (Omniscript® , Marke der QIAGEN GmbH, Hilden, Deutschland) enthielt. Zudem wurden unterschiedliche doppelstrangspezifische Nucleasen zugegeben:
(1) Exonuclease III (E.coli) ("Exo III");
(2) Lambda-Exonuclease (Lamda-Phage) ("LambdaEXO"), und
(3) RNase-freie DNase 1 ("DNase I").

Die doppelstrangspezifischen Nucleasen (1), (2) und (3) wurden jeweils in einer Menge von 10 U (aus Rinderpankreas; erhältlich von Roche, Mannheim. Deutschland) verwendet. Einem weiteren Reaktionsansatz wurde keine Nuklease hinzugefügt. Dieser Ansatz diente als Positiv-Kontrolle. Ferner wurde einem Ansatz Exonuclease VII ("Exo VII") hinzugefügt. Dieser Ansatz diente als Negativkontrolle. Exonuclease VII ist nicht doppelstrangspezifisch und in der Lage, einzelstängige DNA abzubauen. Die jeweiligen Reaktionsgemische wurden für 1 Stunde bei 37°C inkubiert und anschließend durch PCR auf cDNA-Degradierung analysiert. In der PCR wurde das gesamte Transkript von β-Aktin amplifiziert.

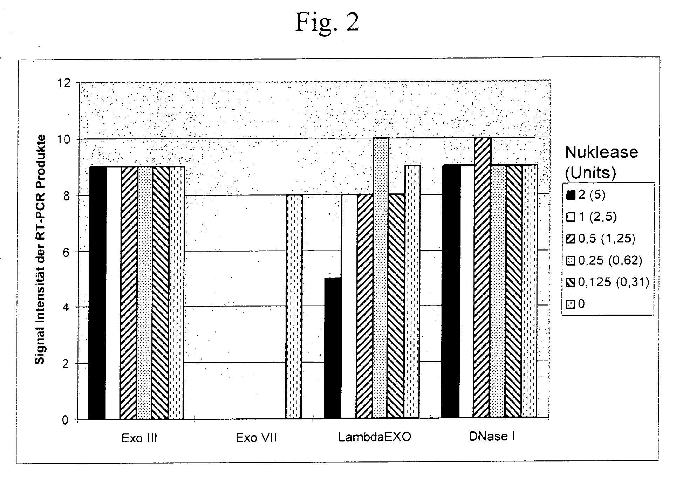

Das Ergebnis ist in Fig. 2 gezeigt. Die drei getesteten Nucleasen (DNase I, Exonuclease III und Lamda-Exonuclease) führten zu keiner Beeinträchtigung der Reverse-Transkriptase Reaktion. Dies ist ersichtlich gegenüber der Positivkontrolle Exo III. Alle drei getesteten doppelstrangspezifischen Nucleasen zeigen eine Signalintensität an RT-PCR-Fragmenten, die der Intensität der Positivkontrolle Exo III sehr nahe kommt. Nur die als Negativkontrolle verwendete einzelstrangspezifische Nuklease Exonuclease VII führte zu einer starken Degradierung der Einzelstrang-cDNA, so daß kein RT-PCR spezifisches Signal gefunden werden konnte.

### Beispiel 3

Jeweils 1 µg hochmolekulare DNA und 1 µg Total-RNA aus HeLa-Zellen wurden gemischt, um in einer Reverse-Transkriptase Reaktion eingesetzt zu werden. Die Reverse-Transkriptase Reaktion wurde dabei in einem wäßrigen Milieu durchgeführt, das einen Oligo-dT-Primer, dNTPs, RNase Inhibitor und einen Puffer (Buffer RT aus dem Omniscript RT Kit der QIAGEN GmbH, Hilden, Deutschland) für die reverse Transkription enthielt. Zudem wurden unterschiedliche Mengen an doppelstrangspezifischen Nucleasen zugegeben:
(1) Alu I Restriktionsendonuklease in einer Menge von 0-10 U (Roche, Mannheim, Deutschland);
(2) Hae III Restriktionsendonuklease in einer Menge von 0-10 U (Roche, Mannheim, Deutschland); und
(3) RNase-freie DNase I in einer Menge von 0-10 U (Roche, Mannheim, Deutschland).

Einem Set der Ansätze wurde Reverse Transkriptase hinzugegeben, um den Einfluß der Nucleasen auf die Synthese einzelsträngiger cDNA untersuchen zu können. Einem zweiten Set von Ansätzen wurde keine reverse Transkriptase hinzugefügt, um die Degradierung der genomischen DNA verfolgen zu können. Die Reaktionsgemische wurden für 1 Stunde bei 37°C inkubiert und anschließend durch PCR analysiert. In den Ansätzen, in denen die cDNA-Synthese verfolgt wurde, wurde die vollständige cDNA des ß-Actin Transkriptes amplifiziert. Bei den Ansätzen, in denen die Degradierung der genomischen DNA verfolgt wurde, wurde ein Bereich aus dem 5'-Ende des ß-Actin Gens amplifiziert. Da das Primerset ein Intron überspannt, zeigt das genomische Amplifikat eine Größe von > 600 bp, während das Amplifikat der cDNA eine Größe von ca. 200 bp aufweist.

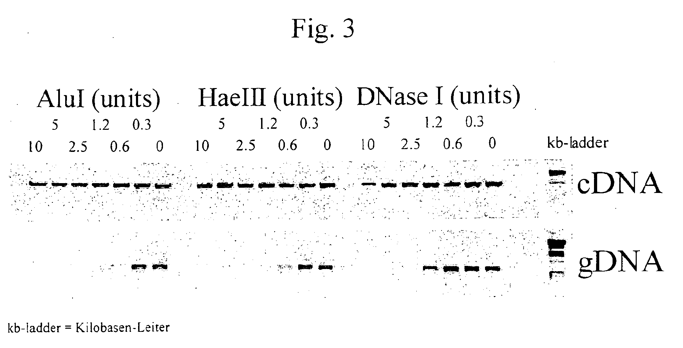

Das Ergebnis ist in Fig. 3 gezeigt. Figur 3 zeigt ein Foto eines 1%-igen Agarosegels auf dessen Bahnen Ansätze mit verschiedenen Nukleasekonzentrationen untersucht wurden. Fig. 3 ist zu entnehmen, daß die Reverse Transkription durch die Anwesenheit der getesteten Nucleasen nicht beeinträchtigt wird, was daran zu erkennen ist, daß die cDNA-Bande für alle drei untersuchten Nucleasen bei allen Konzentrationen klar sichtbar bleibt, siehe den oberen Bereich von Fig. 3. Dagegen ist ebenso klar erkennbar, daß die Verwendung der Nucleasen zu einer mehr oder weniger vollständigen Degradierung der eingesetzten genomischen DNA führt, wenn eine bestimmte Mindestmenge an Nuklease (5 U) zugesetzt wird, siehe unterer Bereich von Fig. 3.

### Beispiel 4

Jeweils 150 ng Total-RNA aus HeLa-Zellen wurden mit 150 ng hochmolekularer DNA gemischt, um eine DNAse-Reaktion unter Reverse Transkriptase Reaktionsbedingungen durchzuführen. Die Reaktion wurde dabei in einem wäßrigen Milieu durchgeführt, das einen Oligo-dT-Primer, dNTPs, RNase Inhibitor, und einen Puffer (Buffer RT aus dem Omniscript RT Kit der QIAGEN GmbH, Hilden, Deutschland)_für die reverse Transkription enthielt. Zudem wurden zu den Ansätzen 0; 0,1; 0,5; oder 2,5 Units doppelstrangspezifische Nuklease zugegeben (RNase-freie DNase I). Zusätzlich wurden zu den Ansätzen 0 mM, 0,5 mM, 1 mM, 1,5 mM, 2 mM oder 2,5 mM Magnesiumchlorid hinzugefügt. Es wurde keine reverse Transkriptase hinzugegeben, um die DNase 1 Aktivität unter Reverse Transkriptase Reaktionsbedingungen zu untersuchen Die Reaktionsgemische wurde für 1 Stunde bei 37°C inkubiert. Anschließend wurde die cDNA Synthese und der DNA-Abbau durch quantitative Echtzeit ("Real Time") PCR analysiert. Es wurden hierzu je 1 µl der Reversen Transkriptase Reaktion für die Real Time PCR eingesetzt. Hierbei wurde ein Primerpaar verwendet, das ein 200 bp Fragment aus dem 5'-Ende des β-Aktin amplifiziert. Das entstandene Amplifikat wurde durch SYBR Green detektiert.

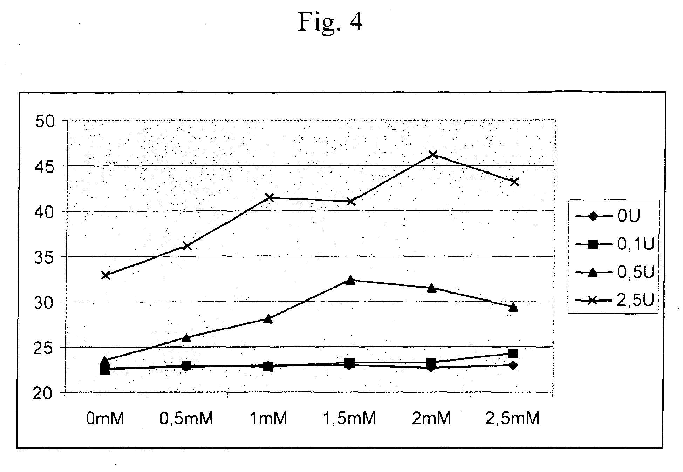

Das Ergebnis ist in Fig. 4 und in Tabelle 1 gezeigt. Der Abbau der genomischen DNA wurde anhand des CT Wertes bestimmt und ist abhängig von der eingesetzten Menge RNase-freier DNase 1. Durch Zugabe von zusätzlichem Magnesiumchlorid kann die Aktivität RNase-freier DNase 1 moduliert werden, wobei für 2,5 U DNase und 2 mM.MgCl₂die höchsten CT-Werte erhalten wurden.

**Tabelle 1**

| MgCl₂ | DNase I (0 U) | DNase I (0,1 U) CT Cyclus | DNase I (0,5 U) CT Cyclus | DNase I (2,5 U) CT Cyclus |
|---|---|---|---|---|
| 0 mM | 22,7 | 22,5 | 23,5 | 32,9 |
| 0,5 mM | 22,8 | 22,9 | 26,1 | 36,2 |
| 1 mM | 22,9 | 22,8 | 28,1 | 41,5 |
| 1,5 mM | 22,9 | 23,3 | 32,3 | 41,1 |
| 2 mM | 22,6 | 23,3 | 31,4 | 46,2 |
| 2,5 mM | 22,9 | 24,3 | 29,4 | 43,2 |

### Beispiele 5

Jeweils 150 ng Total-RNA aus HeLa-Zellen wurden mit 0 ng oder 150 ng hochmolekularer DNA und gemischt, um in einer Reverse-Transkriptase Reaktion eingesetzt zu werden. Die Reaktion fand dabei in einem wäßrigen Milieu statt, das einen Oligo-dT-Primer, dNTPs, RNase Inhibitor und einen Puffer (Buffer RT aus dem Omniscript RT Kit der QIAGEN GmbH, Hilden, Deutschland) für die reverse Transkription enthielt. Zudem wurden zu einem Teil der Ansätze 2,5 Units doppelstrangspezifische Nuklease zugegeben (RNase-freie DNase I). Einem Set der Ansätze wurde reverse Transkriptase hinzugegeben, um den Einfluß der Nucleasen auf die Synthese einzelsträngiger cDNA untersuchen zu können. Einem zweiten Set von Ansätzen wurde keine reverse Transkriptase hinzugegeben, um die Degradierung der genomischen DNA verfolgen zu können. Die Reaktionsgemische wurden für 1 Stunde bei 37°C inkubiert. Anschließend wurde die cDNA-Synthese und der DNA-Abbau durch quantitative Real Time PCR analysiert. Es wurden je 1 µl und 0,1 µl der Reversen Transkriptase-Reaktion für die Real Time PCR eingesetzt. Hierbei wurde ein Primerpaar verwendet, das ein 210 bp Fragment aus dem 3'-Ende des β-Aktin amplifiziert. Das entstandene Amplifikat wurde durch SYBR Green detektiert.

Das Ergebnis dieses Versuches war, daß die reverse Transkription durch die Anwesenheit von RNase-freier DNase I nicht beeinträchtigt wird. Durch die Verwendung von RNase-freier DNase I wird die genomische DNA mehr als 1000-fach abgereichert. Gleichzeitig wird die generierte cDNA nicht oder nur unwesentlich verdaut. Das Ergebnis ist in Tabelle 2 zusammengefaßt.

Der DNase-Schritt kann auch in einer sehr kurzen Reaktion vor der eigentlichen RNAverändernden Reaktion durchgeführt werden, wobei die DNase allerdings wie in den obigen Ansätzen in dem Reaktionsgemisch verbleibt und nicht durch Hitzeinaktivierung oder einen Aufreinigungsschritt aus dem System entfernt wird.

**Tabelle 2**

| **Übertragenes Volumen** | **CT Cyclus 150 ng Hela DNA** | **Mittelwert** | **CT Cyclus 0 ng Hela DNA** | **Mittelwert** |
|---|---|---|---|---|
| **Ohne reverse Transkription, ohne DNAse 1µl** | 22,1 | 22,2 | 32,6 | 33,4 |
| | 22,4 | | 33,5 | |
| | 22,0 | | 34,0 | |
| **Ohne reverse Transkription, ohne DNAse 1µl** | 32,9 | 32,2 | 34,0 | 33,5 |
| | 31,8 | | 35,2 | |
| | 31,8 | | 31,3 | |
| **Ohne reverse Transkription, ohne DNAse 1µl** | 25,3 | 25,4 | 34,4 | 33,2 |
| | 25,4 | | 32,4 | |
| | 25,6 | | 32,9 | |
| **Ohne reverse Transkription, ohne DNAse 1µl** | 34,3 | 34,0 | 32,6 | 32,4 |
| | 33,8 | | 31,5 | |
| | 33,9 | | 33,1 | |
| **Mit reverser Transkription, ohne DNAse 1µl** | 13,3 | 13,4 | 13,4 | 13,4 |
| | 13,5 | | 13,3 | |
| | 13,4 | | 13,5 | |
| **Mit reverser Transkription, ohne DNAse 1µl** | 13,6 | 13,8 | 13,8 | 13,7 |
| | 13,9 | | 13,5 | |
| | 13,8 | | 13,7 | |
| **Mit reverser Transkription, ohne DNAse 1µl** | 16,3 | 16,5 | 15,9 | 16,3 |
| | 16,5 | | 16,4 | |
| | 16,6 | | 16,7 | |
| **Mit reverser Transkription, ohne DNAse 1µl** | 16,0 | 16,3 | 16,2 | 16,5 |
| | 16,5 | | 16,6 | |
| | 16,3 | | 16,6 | |

### Beispiel 6

Jeweils 10 pg bis 1 µg Total-RNA aus HeLa-Zellen wurden mit identischen Mengen hochmolekularer DNA gemischt, um in einer Reverse Transkriptase-Reaktion eingesetzt zu werden. Die Reaktion fand in einem wäßrigen Milieu statt, das einen Oligo-dT-Primer, random Oktamere, dNTPs, RNase Inhibitor und einen Puffer (gDNA Wipeout Buffer und Quantiscript RT Buffer aus dem QuantiTect® Reverse Transcription Kit der QIAGEN GmbH, Hilden, Deutschland)_für die reverse Transkription enthielt. Zudem wurden zu einem Teil der Ansätze 2,5 Units doppelstrangspezifische Nuklease zugegeben (RVase-freie DNase I). Einem Set der Ansätze wurde reverse Transkriptase hinzugefügt, um den Einfluß der Nucleasen auf die Synthese einzelsträngiger cDNA untersuchen zu können. Einem zweiten Set von Ansätzen wurde keine reverse Transkriptase hinzugegeben, um die Degradierung der genomischen DNA verfolgen zu können. Vor der eigentlichen cDNA Synthese wurde der DNase-Schritt für 2 min bei 37°C durchgeführt. Erst danach wurden die Reaktionsgemische für 15 min in Anwesenheit der Reversen Transkriptase bei 37°C inkubiert. Anschließend wurde die cDNA-Synthese und der DNA-Abbau durch quantitative Real Time PCR analysiert. Es wurden je 1 µl der Reversen Transkriptase-Reaktion für die Real Time PCR eingesetzt. Hierbei wurde ein QuantiTect Gene Expression Assay (QIAGEN GmbH, Hilden, Deutschland)) für das Gen RPSLA verwendet, zusammen mit dem QuantiTect Probe PCR Kit (ebenfalls von QIAGEN) verwendet, der alle benötigten Reaktionskomponenten wie HotStarTaq DNA Polymerase, Reaktionspuffer und dNTPs enthält. Die HotStarTaq DNA Polymerase wurde für 15 min bei 95°C reaktiviert, Darauf folgend wurden die PCR Reaktion für 50 Zyklen mit folgendem Temperaturprofil durchgeführt: 15 sec 56 °C, 30 sec 76°C, 30 sec 94°C.. Vor dem Einsatz in der PCR-Reaktion wurde die Reverse Transkriptase-Reaktion für 5 min bei 95°C inaktiviert. Das Maß der genomischen DNA Abreicherung wird in der folgenden Tabelle 3 in CT-Werten angegeben:

**Tabelle 3**

| Differenz -DNase/+DNase | | | | | | |
|---|---|---|---|---|---|---|
| | 1 µg | 100 ng | 10 ng | 1 ng | 100 pg | 10 pg |
| RNA 1 / DNA 1 | 20,6 | 18,7 | 18,1 | 17,8 | 14,2 | 5,4 |
| RNA 2 / DNA 2 | 23,3 | 20,7 | 20,0 | 13,6 | 8,3 | 10,4 |
| RNA 2 / DNA 1 | 23,1 | 21,7 | 21,4 | 18,8 | 14,8 | 12,1 |

Das Ergebnis zeigt, daß durch die Verwendung von RNase-freier DNase I, deren Inkubation dem eigentlichen Reverse Transkriptase-Schritt im Reaktionsansatz vorgeschaltet ist, die genomische DNA in der Regel mehr als 1000-fach abgereichert wird.

In einem weiteren Ansatz sollte gezeigt werden, daß der DNase-Schritt auch in den Prozeß einer sogenannten one-step-RT-PCR integriert werden kann. Bei einer one-step-RT-PCR Reaktion wird der gesamte Reaktionsansatz inklusive aller benötigten Reagenzien für den Reverse Transkriptase-Schritt und den anschließenden PCR Schritt zusammengefügt. Die Reaktion wird mit der Reversen Transkription gestartet und geht direkt ohne Öffnen des Reaktionsgefäßes in den PCR-Schritt über. Das folgende Beispiel zeigt, daß ein DNase-Schritt auch in ein solches durchgängiges Verfahrensschema, das keine weiteren Anwenderinteraktionen zuläßt, eingefügt werden kann.

### Beispiel 7

Jeweils 20 ng total RNA aus HeLa Zellen und 20 ng hochmolekulare gDNA wurden in jeder one-step-RT-PCR-Reaktion eingesetzt. Der Reaktion wurden 150 µM CaCl₂ zugesetzt. Jede Reaktion wurde mit dem QuantiTect RT-PCR Kit (QIAGEN GmbH, Hilden, Deutschland) durchgeführt, welcher alle benötigten Reaktionskomponenten wie Reverse Transkriptase, HotStarTaq DNA Polymerase, Reaktionspuffer und dNTPs enthält. Die Reaktionen wurden mit und ohne DNase I angesetzt. In Reaktionen, die ausschließlich genomische DNA nachweisen sollten, wurde die Reverse Transkriptase nicht eingesetzt, um kein zusätzliches Signal von einer cDNA zu erhalten. Reaktionsansätze, in denen DNase I eingesetzt wurde, enthielten 0,25 units DNase I. Als Zielgen wurde ein Transkriptbereich nachgewiesen, für das in der genomischen DNA identische Sequenzen vorkommen. Die PCR-Produkte, die aus genomischer DNA und cDNA generiert wurden, hatten die gleiche Größe und sollten demzufolge mit gleicher Effizienz amplifiziert und nachgewiesen werden.

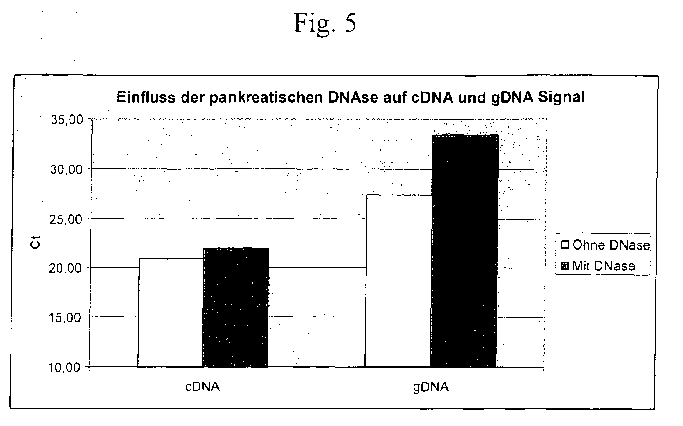

Das Ergebnis ist in Fig. 5 gezeigt und läßt sich wie folgt zusammenfassen. Der CT Wert der genomischen DNA vergrößert sich durch Einsatz der DNase um ca. 6 Zyklen, was einer hundertfachen gDNA-Abreicherung entspricht, während sich der CT Wert der cDNA nur unwesentlich verändert. Dies läßt die Schlußfolgerung zu, daß ein gDNA-Entfernungsschritt auch in einer one-step-RT-PCR einsetzbar ist und zu signifikanten Abreicherungen der genomischen DNA führt, während die cDNA intakt bleibt oder nur unwesentlich degradiert wird. Die Erhöhung des CT Wertes bei Verwendung von RNA (cDNA) ist im wesentlichen auf die Abreicherung der in der RNA Probe enthaltenen genomischen DNA zurückzuführen.

## Patentansprüche

1. RNA-Reaktionsverfahren, **dadurch gekennzeichnet, daß** im selben Reaktionsgefäß zur gleichen Zeit und bei der selben Temperatur eine RNA-Reaktion und eine Degradierung von vorhandener DNA erfolgt.

2. RNA-Reaktionsverfahren nach Anspruch 1, **dadurch gekennzeichnet, daß** die RNA-Reaktion eine Markierung, Verlängerung und/oder Modifizierung der RNA ist.

3. RNA-Reaktionsverfahren nach Anspruch 1, **dadurch gekennzeichnet, daß** die RNA-Reaktion eine spezifische Degradierung ist, bei der RNA selektiv degradiert wird, beispielsweise durch Ribozyme, RNase H und/oder bei siRNA-induzierter RNA-Degradierung.

4. RNA-Reaktionsverfahren nach Anspruch 1, **dadurch gekennzeichnet, daß** die RNA-Reaktion eine Umsetzung ist, bei der RNA als Matrize für Polymerasereaktionen verwendet wird.

5. RNA-Reaktionsverfahren nach Anspruch 4, **dadurch gekennzeichnet, daß** die Umsetzung eine reverse Transkription ist.

6. RNA-Reaktionsverfahren nach einem der Ansprüche 1 bis 4, **dadurch gekennzeichnet, daß** RNA bei der RNA-Reaktion als Katalysator wirkt.

7. RNA-Reaktionsverfahren nach einem der Ansprüche 1 bis 6, **dadurch gekennzeichnet, daß** RNA bei der RNA-Reaktion an einer Bindungsreaktion beteiligt ist.

8. RNA-Reaktionsverfahren nach einem der Ansprüche 1 bis 7, **dadurch gekennzeichnet, daß** die RNA-Reaktion eine zusammengesetzte Reaktion ist, an der mindestens zwei der in den Ansprüchen 2 bis 5 genannten Reaktionen beteiligt sind.

9. RNA-Reaktionsverfahren nach Anspruch 4, **dadurch gekennzeichnet, daß** unter der Voraussetzung, dass die verwendete Reverse Transkriptase eine RNase H-Aktivität aufweist (RNase H+), vor dem Schritt, in dem eine RNA-Reaktion und eine Degradierung von vorhandener DNA im selben Reaktionsgefäß zur gleichen Zeit und bei der selben Temperatur gemäß Anspruch 1 erfolgt, durch eine reine Temperaturänderung ein weiterer Schritt durchgeführt wird, bei welchem die Temperatur soweit verringert wird, dass die Degradierung von DNA erfolgt, die RNase H-Aktivität der Reversen Transkriptase jedoch unterdrückt wird.

10. RNA-Reaktionsverfahren nach einem der Ansprüche 1 bis 9, **dadurch gekennzeichnet, daß** die Degradierung von DNA mittels eines DNA-Einzelstrang-spezifischen Enzyms und/oder mittels eines DNA-Doppelstrang-spezifischen Enzyms erfolgt.

11. RNA-Reaktionsverfahren nach Anspruch 10, **dadurch gekennzeichnet, daß** die Degradierung von DNA mittels eines Enzyms ausgewählt aus der Gruppe von Exo III. Lambda Exo, DNase I und Restriktionsendonukleasen erfolgt.

12. RNA-Reaktionsverfahren nach einem der Ansprüche 1 bis 11, **dadurch gekennzeichnet, daß** die Reaktion bei einem pH-Wert zwischen 6 und 10, vorzugsweise zwischen 7 und 9, durchgeführt wird.

13. RNA-Reaktionsverfahren nach einem der Ansprüche 1 bis 12, **dadurch gekennzeichnet, daß** der Reaktionsansatz bivalente Kationen enthält.

14. RNA-Reaktionsverfahren nach Anspruch 13, **dadurch gekennzeichnet, daß** die bivalenten Kationen Mg²⁺, Mn²⁺ und/oder Ca²⁺ sind.

15. Kit zur Durchführung einer cDNA Synthesereaktion, insbesondere zur Durchführung eines Verfahrens gemäß einem der Ansprüche 1 bis 12, der Kit enthaltend mindestens eine reversen Transkriptase, eine DNA abhängige Nuclease und einen Reaktionspuffer zur Durchführung einer cDNA Synthese und einer DNA Degradierung in einem Gefäß.

16. Kit zur Durchführung einer 1-step-RT-PCR Reaktion, insbesondere zur Durchführung eines Verfahrens gemäß einem der Ansprüche 1 bis 12. der Kit enthaltend mindestens eine reverse Transkriptase, eine DNA abhängige Nuclease, eine thermostabile DNA Polymerase und einen Reaktionspuffer zur Durchführung einer 1-step-RT PCR Reaktion und einer DNA Degradierung in einem Gefäß.
